(19) Europäisches Patentamt
European Patent Office
Office européen des brevets

(11) **EP 3 753 484 B1**

(12) **EUROPEAN PATENT SPECIFICATION**

(45) Date of publication and mention
of the grant of the patent:
**08.03.2023  Bulletin 2023/10**

(21) Application number: **20170753.6**

(22) Date of filing: **22.04.2020**

(51) International Patent Classification (IPC):
***A61B 5/021*** (2006.01)

(52) Cooperative Patent Classification (CPC):
**A61B 5/02108;** A61B 2560/0223

(54) **APPARATUS AND METHOD FOR CALIBRATING BIO-INFORMATION ESTIMATION MODEL, AND APPARATUS FOR ESTIMATING BIO-INFORMATION**

VORRICHTUNG UND VERFAHREN ZUR KALIBRIERUNG EINES BIOINFORMATIONSSCHÄTZUNGSMODELLS UND VORRICHTUNG ZUM SCHÄTZEN VON BIOINFORMATIONEN

APPAREIL ET PROCÉDÉ D'ÉTALONNAGE D'UN MODÈLE D'ESTIMATION D'INFORMATIONS BIOLOGIQUES ET APPAREIL D'ESTIMATION D'INFORMATIONS BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **19.06.2019   KR 20190072661**

(43) Date of publication of application:
**23.12.2020   Bulletin 2020/52**

(73) Proprietor: **Samsung Electronics Co., Ltd.
Gyeonggi-do 16677 (KR)**

(72) Inventors:
• **JANG, Dae Geun
Gyeonngi-do (KR)**
• **KWON, Ui Kun
Gyeonngi-do (KR)**
• **PARK, Chang Soon
Chungcheongbuk-do (KR)**
• **YOON, Seung Keun
Seoul (KR)**

(74) Representative: **Grünecker Patent- und Rechtsanwälte
PartG mbB
Leopoldstraße 4
80802 München (DE)**

(56) References cited:
EP-A1- 3 130 280        US-A1- 2011 021 929
US-A1- 2017 209 053     US-A1- 2018 360 323

**Description**

BACKGROUND

1. Field

The disclosure relates to a technology for calibrating a bio-information estimation model, and estimating bio-information based on the calibrated bio-information estimation model.

2. Description of Related Art

Generally, methods of non-invasively measuring blood pressure without damaging a human body include a method of measuring blood pressure by measuring a cuff-based pressure, and a method of estimating blood pressure by measuring a pulse wave without the use of a cuff. A Korotkoff-sound method is a cuff-based blood pressure measurement method, in which a pressure in a cuff wound around an upper arm is increased and blood pressure is measured by listening to the sound generated in the blood vessel through a stethoscope while decreasing the pressure. Another cuff-based blood pressure measurement method is an oscillometric method using an automated machine, in which a cuff is wound around an upper arm, a pressure in the cuff is increased, a pressure in the cuff is continuously measured while the cuff pressure is gradually decreased, and blood pressure is measured based on a point where a change in a pressure signal exceeds a threshold. Cuff-less blood pressure measurement methods generally include a method of measuring blood pressure by calculating a pulse transit time (PTT) and a pulse wave analysis (PWA) method of estimating blood pressure by analyzing a shape of a pulse wave.

EP 3130280 A1 discloses a blood pressure estimating method including measuring a biosignal including pulse wave information of a user, determining a calibration method for a blood pressure estimation model, calibrating the blood pressure estimation model using the determined calibration method, and estimating a blood pressure of the user from the biosignal using the calibrated blood pressure estimation model. The method comprises determining whether to calibrate the blood pressure estimation model based on either one or both of a signal quality of the biosignal and an elapsed time since a previous calibration of the blood pressure estimation model was performed.

SUMMARY

The invention is defined in claims 1, 6 and 7. Preferred embodiments of the invention are defined in the dependent claims.

Additional aspects of the disclosure will be set forth in part in the description which follows and, in part, will be apparent from the description, or may be learned by practice of the presented embodiments.

In accordance with an aspect of the disclosure, an apparatus for calibration of a bio-information estimation model may include a sensor configured to detect a bio-signal from an object of interest, and a processor configured to extract a first feature at each of a plurality of points of the bio-signal, extract a second feature at each of the plurality of points of the bio-signal, and determine a calibration point of the bio-information estimation model, from among the plurality of points of the bio-signal, based on a first degree of change in the first feature at each point relative to a first feature at a reference point and a second degree of change in the second feature at each point relative to a second feature at the reference point.

The bio-signal may include at least one of a photoplethysmogram (PPG) signal, an impedance plethysmography (IPG) signal, a video plethysmography (VPG) signal, an electrocardiography (ECG) signal, an electromyography (EMG) signal, and a ballistocardiogram (BCG) signal. According to the claimed invention, the bio-signal includes a pulse wave signal.

The bio-signal may include a single bio-signal measured during a single time period or a plurality of bio-signals measured during a plurality of time periods.

According to the claimed invention, the processor sets each of the plurality of points of the bio-signal as the reference point in turn, and calculate the first degree of change in the first feature and the second degree of change in the second feature at each of the plurality of points relative to each set reference point.

The processor may combine the first degree of change in the first feature and the second degree of change in the second feature which are calculated at each point relative to each reference point, and determine a reference point at which the combination result satisfies a predetermined criterion as the calibration point.

According to the claimed invention, the processor calculates a sum of residual between the first degree of change in the first feature and the second degree of change in the second feature at each point for each reference point, and determine a reference point at which the calculated sum of residual has a minimum value as the calibration point.

The processor may determine two or more valid points from among the plurality of points, and determine the calibration point from among the determined valid points.

The processor may determine the valid points based on a correlation between the first degree of change in the first feature and the second degree of change in the second feature at each point of the plurality of points of the bio-signal.

The processor may acquire representative waveforms corresponding to each of the points by ensemble averaging a waveform of the bio-signal in units of a predetermined number of bits, and extract the first feature and the second feature from each of the representative waveforms.

In accordance with an aspect of the disclosure, an apparatus for calibration of a bio-information estimation model may include a sensor configured to detect a bio-signal from an object of interest, and a processor configured to extract a first feature from a plurality of points of the bio-signal, extract a second feature from the plurality of points of the bio-signal, and determine a coupling coefficient of the bio-information estimation model based on a first degree of change in the first feature at each point relative to a first feature at a calibration point and a second degree of change in the second feature at each point relative to a second feature at the calibration point.

The processor may set a single pre-set point from the plurality of points of the bio-signal as the calibration point.

The processor may set each of the plurality of points as a reference point in turn, and determine the calibration point from the plurality of points of the bio-signal based on a first degree of change in the first feature at each point relative to a first feature at the reference point and a second degree of change in the second feature at each point relative to a second feature at the reference point.

The processor may determine the coupling coefficient such that a result of combining the first degree of change in the first feature and the second degree of change in the second feature at each point by applying the coupling coefficient to one of the first degree of change in the first feature and the second degree of change in the second feature satisfies a predetermined criterion.

The processor may determine the coupling coefficient such that a sum of residual between the first degree of change in the first feature and the second degree of change in the second feature has a minimum value.

A range of the coupling coefficient may be pre-set based on one or more of a user input, a user characteristic, an external environment characteristic, a bio-information estimation history, a type of bio-information to be estimated, and a computing performance metric of the apparatus.

The processor may determine two or more valid points from among the plurality of points, and determine the coupling coefficient based on respective degrees of change in a first feature and a second feature at the determined valid points.

In accordance with an aspect of the disclosure, a method of calibration of a bio-estimation model includes detecting a bio-signal from an object of interest, extracting a first feature from each of a plurality of points of the bio-signal, extracting a second feature from each of the plurality of points of the bio-signal, identifying a first degree of change in the first feature at each point relative to a first feature at a reference point, identifying a second degree of change in the second feature at each point relative to a second feature at the reference point, and determining a calibration point of the bio-information estimation model from among the plurality of points of the bio-signal based on the first degree of change in the first feature and the second degree of change in the second feature.

According to the claimed invention, the identifying of the first degree of change in the first feature and the second degree of change in the second feature respectively comprises setting each of the plurality of points as the reference point in turn and calculating the first degree of change in the first feature and the second degree of change in the second feature at each point relative to each reference point.

The determining of the calibration point may comprise combining the first degree of change in the first feature and the second degree of change in the second feature at each point relative to each reference point and determining a reference point at which the combination result satisfies a predetermined criterion as the calibration point.

According to the claimed invention, the determining of the calibration point comprises calculating a sum of residual between the first degree of change in the first feature and the second degree of change in the second feature at each point for each reference point and determining a reference point at which the calculated sum of residual has a minimum value as the calibration point.

The determining of the calibration point may comprise determining two or more valid points from among the plurality of points and determining the calibration point from among the determined valid points.

The determining of the calibration point may comprise determining the valid points based on a correlation between the first degree of change in the first feature and the second degree of change in the second feature at each point.

In accordance with an aspect of the disclosure, a method of calibration of a bio-information estimation may include detecting a bio-signal from an object of interest, extracting a first feature from a plurality of points of the bio-signal, extracting a second feature from the plurality of points of the bio-signal, determining a calibration point from among the plurality of points of the bio-signal, identifying a first degree of change in a first feature at each point relative to a first feature at the calibration point, identifying a second degree of change in a second feature at each point relative to a second feature at the calibration point, and determining a coupling coefficient of the bio-information estimation model based on the first degree of change in the first feature and the second degree of change in the second feature.

The determining of the calibration point may comprise determining a single pre-set point from the plurality of points as the calibration point.

The determining of the calibration point may comprise setting each of the plurality of points as a reference point in turn and determining the calibration point from the plurality of points based on a degree of change in the first feature at each point relative to a first feature at the reference point and a degree of change in the second feature at each point relative to a second feature at the reference point.

The determining of the coupling coefficient may comprise determining the coupling coefficient such that a result of combining the first degree of change in the first feature and the second degree of change in the second feature at each point by applying the coupling coefficient to one of the first degree of change in the first feature and the second degree of change in the second feature satisfies a predetermined criterion.

The determining of the coupling coefficient may comprise determining the coupling coefficient such that a sum of residual between the first degree of change in the first feature and the second degree of change in the second feature has a minimum value.

The determining of the coupling coefficient may comprise determining two or more valid points from among the plurality of points and determining the coupling coefficient based on respective degrees of change in a first feature and a second feature at the determined valid points.

In accordance with an aspect of the disclosure, an apparatus for estimating bio-information may include a sensor configured to detect a first bio-signal and a second bio-signal from an object of interest, and a processor configured to extract a plurality of features from each of a plurality of points of the first bio-signal, determine a calibration point from among the plurality of points based on a degree of change in each feature at each point relative to a reference point, calibrate the bio-information estimation model based on a feature at the determined calibration point, and estimate the bio-information based on the second bio-signal and the calibrated bio-information estimation model.

The processor may set each of the plurality of points as a reference point in turn, calculate a sum of residual between the respective degrees of change in the features at each point relative to each set reference point, and determine a reference point at which the calculated sum of residual has a minimum value as the calibration point.

The processor may extract the plurality of features from the second bio-signal, and estimate the bio-information using the calibrated bio-information estimation model based on a degree of change in each feature extracted from the second bio-signal relative to each feature at the calibration point.

The bio-information includes at least one of blood pressure, vascular age, a degree of arteriosclerosis, an aortic pressure waveform, vascular compliance, a stress index, and a degree of fatigue.

Other features and aspects will be apparent from the following detailed description, the drawings, and the claims.

## BRIEF DESCRIPTION OF THE DRAWINGS

The above and other aspects, features, and advantages of certain embodiments of the present disclosure will be more apparent from the following description taken in conjunction with the accompanying drawings, in which:

FIG. 1 is a block diagram illustrating an apparatus for calibrating a bio-information estimation model according to an embodiment.

FIG. 2 is a block diagram illustrating a configuration of the processor of FIG. 1 according to an embodiment.

FIGS. 3A to 3D are graphs for describing calibration according to an embodiment.

FIG. 4 is a block diagram illustrating a configuration of the processor of FIG. 1 according to an embodiment.

FIG. 5 is a graph for describing calibration according to an embodiment.

FIG. 6 is a flowchart illustrating a method of calibration according to an embodiment.

FIG. 7 is a flowchart illustrating a method of calibration according to an embodiment.

FIG. 8 is a block diagram illustrating an apparatus for estimating bio-information according to an embodiment.

FIG. 9 is a diagram illustrating a wearable device according to an embodiment.

FIG. 10 is a diagram illustrating a smart device according to an embodiment.

Throughout the drawings and the detailed description, unless otherwise described, the same drawing reference numerals may refer to the same elements, features, and structures. The relative size and depiction of these elements may be exaggerated for clarity, illustration, and convenience.

## DETAILED DESCRIPTION

The embodiments are described in greater detail below with reference to the accompanying drawings.

In the following description, like drawing reference numerals may be used for like elements, even in different drawings. The matters defined in the description, such as detailed construction and elements, are provided to assist in a comprehensive understanding of the embodiments. However, it is apparent that the exemplary embodiments can be practiced without those specifically defined matters. Also, well-known functions or constructions might not be described in detail so as to not obscure the description.

It will be understood that, although terms such as "first," "second," etc., may be used herein to describe various elements, these elements might not be limited by these terms. These terms may be used to distinguish one element from another. Also, the singular forms of terms may include the plural forms of the terms as well, unless the context clearly indicates otherwise. In the specification, unless explicitly described to the contrary, the term "comprise" and variations such as "comprises" or "comprising," will be understood to imply the inclusion of the stated elements but not the exclusion of any other elements. Terms such as "unit," "module," "component," etc., may denote units, modules, components, etc., that process at least one function or operation, and the units, modules, components, etc., may be implemented in hardware, software, or a combination of hardware and software.

Hereinafter, an apparatus and method for calibrating a bio-information estimation model and an apparatus for estimating bio-information will be described with reference to the accompanying drawings.

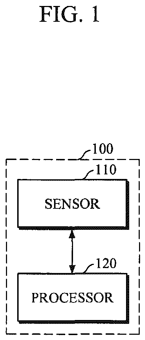

FIG. 1 is a block diagram illustrating an apparatus for calibrating a bio-information estimation model according to an embodiment. The bio-information estimation model may be a model that is implemented in various forms, such as a linear/nonlinear mathematical function, for estimating blood pressure, vascular age, a degree of arteriosclerosis, an aortic pressure waveform, vascular compliance, a stress index, a degree of fatigue, and/or the like. Hereinafter, and as an example, the bio-information model may be described as being configured to measure blood pressure for convenience of description.

Referring to FIG. 1, the apparatus 100 for calibration includes a sensor 110 and a processor 120.

The sensor 110 may be configured to detect a bio-signal of an object of interest of a user under the control of the processor 120. In this case, the object of interest may be human skin tissue, and may be, for example, a human body part, such as a back of a hand, a wrist, a finger, or the like, under which capillary blood or venous blood passes. However, the example of the object of interest is not limited to the above examples, and may be a human body part where an artery, such as a radial artery, passes. In this case, the bio-signal may include a photoplethysmogram (PPG) signal, an impedance plethysmography (IPG) signal, a video plethysmography (VPG) signal, an electrocardiography (ECG) signal, an electromyography (EMG) signal, a ballistocardiogram (BCG) signal, and the like. However, the examples of the bio-signal are not limited to the above examples.

For example, the sensor 110 may include a pulse wave sensor that is configured to detect a pulse wave signal including a PPG signal. The pulse wave sensor may include a light source configured to emit light towards the object of interest, and a detector configured to detect light scattered or reflected by a living tissue, such as a skin surface or a blood vessel of the object of interest irradiated by the light source.

The light source may emit light of a predetermined wavelength band towards the object of interest that is in contact with the pulse wave sensor. Here, the predetermined wavelength band may be an infrared band, a visible light band, and the like. The light source may include a light emitting diode (LED), a laser diode (LD), a phosphor, and the like. However, the light source is not limited to the above examples. The light source may be configured as a single LED, and may emit light of a single wavelength. Alternatively, the light source may be configured as an array of a plurality of LEDs, and each of the LEDs may emit light of the same wavelength, or at least some LEDs may emit light of different wavelengths from each other.

The detector may detect light reflected by the tissue of the object interest which absorbs, scatters, or reflects the light emitted from the light source, and may convert the intensity of the detected light into an electrical signal and output the electrical signal. The detector may include a photodiode, a photo transistor (PTr), an image sensor (e.g., complementary metal-oxide-semiconductor (CMOS) image sensor), or the like. However, the detector is not limited to the above examples. For example, the detector may be configured with a single photodiode or a plurality of photodiode arrays. In this case, each of the detectors may be disposed at a different distance from the light source and may detect light scattered or reflected from the tissue of the object of interest irradiated by the light source at a different position.

Based on a calibration event, the processor 120 may control the sensor 110 to detect a bio-signal for calibration. The processor 120 may control an output component to provide the user with guide information for maintaining a stable state and measuring a bio-signal so that a bio-signal can be accurately detected from the object of interest in a stable state of the user. For example, the processor 120 may control an output component to provide information regarding a contact position and/or a contact pressure between the object of interest and the pulse wave sensor so that the user can accurately contact the object of interest with the pulse wave sensor.

For example, the processor 120 may analyze a bio-signal detected during a time period by the sensor 110, and calibrate the bio-information estimation model.

In another example, the processor 120 may perform calibration using a plurality of bio-signals detected during a plurality of time periods. For instance, during calibration, the processor 120 may control the sensor 110 to detect a plurality of bio-signals at predetermined time intervals. In this case, the time interval for measuring a bio-signal may be preset by taking into consideration the time taken by the user to return to a stable state after measuring a bio-signal in a stable state. Alternatively, a bio-signal detected during a current calibration, and a bio-signal detected during a previous calibration may be obtained.

The processor 120 may communicate with an external device by controlling a communication module, and may receive information that serves as reference for calibration of bio-information from the external device. Additionally, the processor 120 may transmit information generated as a result of calibration such as, for example, a reference feature and a bio-information estimation model calibrated with a coupling coefficient, which will be described below, to the external device, such as an apparatus for estimating bio-information. In this case, the external device may include a blood pressure measurement apparatus, a medical device related to measurement of other bio-information, and an information processing apparatus, such as a smartphone, a tablet personal computer (PC), a desktop PC, a notebook PC, and the like.

The communication module may use various wired/wireless communication technologies, such as Bluetooth communication, Bluetooth low energy (BLE) communication, near field communication (NFC), wireless local area network (WLAN) communication, ZigBee communication, infrared data association (IrDA) communication, wireless fidelity (Wi-Fi) communication, ultra-wideband (UWB) communication, Ant+ communication, Wi-Fi direct (WFD) communication, radio frequency identification (RFID) communication, third generation (3G) communication, fourth generation (4G) communication, fifth generation (5G) communication, and the like.

The processor 120 may control an output component to provide a processing result to the user. For example, information generated during calibration, for example, information regarding a reference feature and/or a coupling coefficient, which will be described below, may be output to the user. In this case, the output component may include a display, a speaker, a haptic component, and the like.

The processor 120 may store information generated as a result of processing in a storage. For example, reference information for calibration, a detected bio-signal, information regarding a reference feature and/or a coupling coefficient which are generated as a result of calibration, calibration history information, and the like, may be stored and managed. In this case, the reference information may include user characteristic information, such as the user's age, sex, health status, etc., and a bio-information estimation model.

The storage may include a non-transitory computer-readable storage medium, such as a flash memory type, a hard disk type, a multimedia card micro type, a card type memory (e.g., secure digital (SD) or extreme digital (XD) memory), random access memory (RAM), static random access memory (SRAM), read-only memory (ROM), electrically erasable programmable read-only memory (EEPROM), programmable read-only memory (PROM), a magnetic memory, a magnetic disk, an optical disk, and the like, but is not limited thereto.

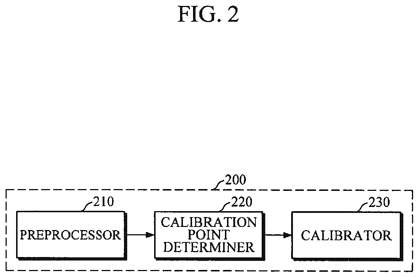

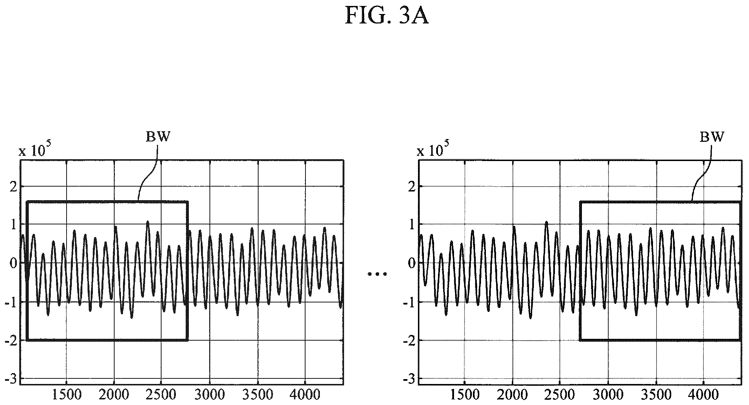

FIG. 2 is a block diagram illustrating a configuration of the processor of FIG. 1. FIGS. 3A to 3D are graphs for describing calibration according to an embodiment.

Referring to FIG. 2, a processor 200 according to an embodiment may include a preprocessor 210, a calibration point determiner 220, and a calibrator 230.

Based on the preprocessor 210 receiving a bio-signal for calibration from the sensor 110, the preprocessor 210 may preprocess the received bio-signal. For example, the preprocessor 210 may perform preprocessing, such as noise removal and signal amplification by detrending in which a bio-signal is normalized and the trend and offset are removed, 0.4-10 Hz band-pass filtering, low-pass filtering, and the like.

In addition, the preprocessor 210 may detect representative waveforms from a plurality of points of the bio-signal. For example, the preprocessor 210 may detect representative waveforms from a waveform of a bio-signal at a predetermined number of points on a time axis. Referring to FIG. 3A, a representative waveform may be detected by calculating an ensemble average of the waveform of the bio-signal in units of a predetermined number of bits. As shown in FIG. 3A, the predetermined number of bits are used as one window bandwidth (BW) and a plurality of representative waveforms may be acquired by sliding the window BW.

The preprocessor 210 may adaptively adjust a window size based on a time period for measuring a bio-signal, the number of representative waveforms to be detected from a bio-signal, a window size (i.e., the number of bits included in a bit unit), and the like.

For example, the preprocessor 210 may adjust the time period for measuring a bio-signal for each user or the number of representative waveforms to be detected based on a user input, a user characteristic (e.g., a health status, sex, age, and the like), an external environment characteristic (e.g., a change in temperature, humidity, season, and the like), bio-information estimation history, a type of bio-information to be estimated, a computing performance of a device, and the like. For example, based on the time period for measuring a bio-signal being reduced or the number of representative waveforms to be detected being increased, the preprocessor 210 may reduce the window size to detect more representative waveforms. However, the embodiment is not limited to the example.

Based on a plurality of bio-signals being acquired, the preprocessor 210 may detect a predetermined number of representative waveforms by detecting one or more representative waveforms from each of the plurality of bio-signals.

Based on the plurality of representative waveforms being detected by the preprocessor 210, the calibration point determiner 220 may extract two or more features from each of the representative waveforms. The calibration point determiner 220 may acquire the features by analyzing each representative waveform and derivative/integrated signals of each representative waveform. In this case, the order of derivative and integration is not particularly limited.

Here, the feature may be a value having a high degree of correlation with actual bio-information, and may be, for example, a value that increases/decreases in the same direction in which the blood pressure increases/decreases. Generally, a pulse wave signal is a summation of a propagation wave propagating from the heart to peripheral parts of a body, and reflection waves returning from the peripheral parts of the body. The calibration point determiner 220 may extract a feature having a high degree of correlation with blood pressure by combining time and amplitude information obtained at a position of each pulse wave component in a pulse wave signal.

For example, the calibration point determiner 220 may extract a shape of each representative waveform, time and amplitude of a maximum point, time and amplitude of a minimum point, time and amplitude at a position of a pulse wave component constituting a bio-signal, an area of one or more regions of a bio-signal, a heartrate, and the like, and acquire a new feature based on a combination of two or more acquired features. In this case, the combination may be made in various ways, such as addition, subtraction, division, multiplication, log value, and combinations thereof, and is not particularly limited to any particular type of combination. In this case, the calibration point determiner 220 may derive a second order derivative signal by second order differentiating each of the representative waveforms in order to acquire positions of pulse wave components constituting each of the pulse waveforms, and may search for a minimum point of the second drive signal as a position of the pulse wave component.

Generally, the amount of change in mean arterial pressure (MAP) is proportional to cardiac output (CO) and total peripheral resistance (TPR) as shown in Equation 1 below.

$$\Delta MAP = CO \times TPR \qquad \text{...Equation (1)}$$

Referring to Equation 1, $\Delta MAP$ represents a difference in MAP between the left ventricle and the right atrium. Because MAP of the right atrium is generally in a range of 3 to 5 mmHg, mean blood pressure (MBP) obtained by Equation 1 is similar to MBP of the left ventricle or MBP of an upper arm. Therefore, if absolute CO and TPR values are known, MAP at the aorta or at the upper arm may be acquired..

To improve the estimation of absolute CO and TPR values, the calibration point determiner 220 may extract a feature associated with CO (hereinafter referred to as a "first feature") and a feature associated with TPR (hereinafter referred to as a "second feature") at each of a plurality of points of the bio-signal. Here, the feature associated with CO may be a feature that shows a tendency of increasing or decreasing proportionally to CO when the CO is relatively increased or decreased by greater than a threshold as compared to the TPR that is substantially constant as compared to a stable state. Similarly, the feature value associated with the TPR may be a feature value that shows a tendency of increasing or decreasing proportionally to TPR when the TPR is relatively increased or decreased by greater than a threshold as compared to the CO that is substantially constant as compared to a stable state.

The calibration point determiner 220 may determine a calibration point based on a correlation between the first feature and the second feature when the first feature and the second feature are extracted at each point.

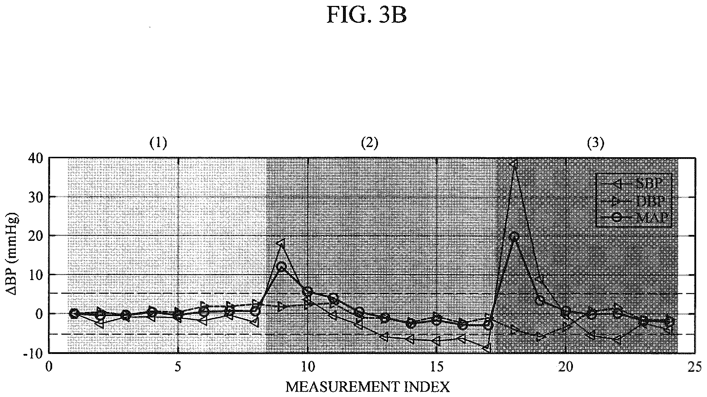

For example, FIG. 3B shows a change in blood pressure of a plurality of users according to various change mechanisms of blood pressure, wherein a first region (1) shows a change in blood pressure measured during a stable state in which exercise is not performed, a second region (2) shows a change in blood pressure measured during a state in which aerobic exercise is performed, and a third region (3) shows a change in blood pressure measured in a state in which anaerobic exercise is performed. Referring to FIG. 3B, it may be assumed that the amount of change in MAP in a stable state (1) is negligible.

Thus, the calibration point determiner 220 may determine a point at which the amount of change of MAP ($\Delta$MAP) calculated using a plurality of first features and second features extracted from a plurality of points of the bio-signal detected during a stable state becomes zero, or a point at which $\Delta$MAP is close to zero as a calibration point.

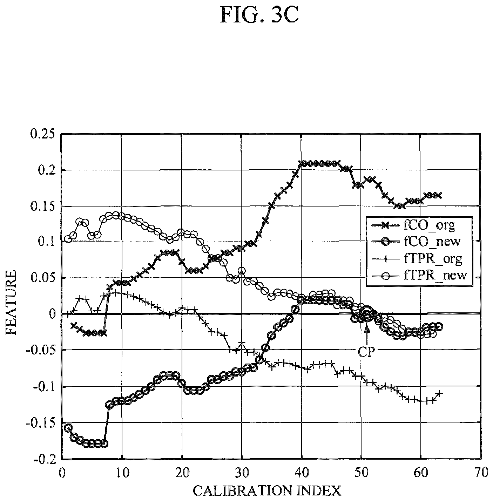

FIG. 3C shows the amount fCO_org of change in the first feature and the amount fTPR_org of change in the second feature which are obtained based on a generally determined calibration point such as, for example, a point at which a calibration index is zero, and a normalized amount fCO_new of change in first feature and a normalized amount fTPR_new of change in second feature which are obtained based on a calibration point CP determined according to the present embodiment.

For example, based on the first feature and the second feature being extracted at each of the plurality of points, the calibration point determiner 220 may calculate degrees of change in the first feature and the second feature at each point relative to the first feature and the second feature at a reference point and normalize the degrees of change in the first and second features. For example, the degree of change in the first feature may be obtained by subtracting the first feature at the reference point from the first feature at each point, and the degree of change may be normalized by being divided by the first feature at the reference point. Similarly, the degree of change in the second feature may be obtained by subtracting the second feature at the reference point from the second feature at each point and the degree of change may be normalized by being divided by the second feature at the reference point.

The calibration point determiner 220 may set each of the plurality of points of the bio-signal as a reference point in turn, and calculate and normalize the degrees of change in the first feature and the second feature at each point, relative to each reference point. In addition, the calibration point determiner 220 may combine the normalized degrees of change in the first feature and the second feature at each point for each reference point, and determine, based on combination results, that a reference point that satisfies a predetermined criterion is a calibration point.

For example, Equation 2 below is one example of combining the degree of change in the first feature and the degree of change in the second feature. A sum of residual between the degree of change in the first feature and the degree of change in the second feature for each reference point may be acquired, and a point where the sum of residual has the minimum value may be determined as a calibration point.

$$SR_j = \sum_{i=1}^{M} \left|\left|\Delta f_{1,i}\right| - \left|\Delta f_{2,i}\right|\right| \qquad \text{...Equation (2)}$$

Referring to Equation 2, $M$ represents the number of points of a bio-signal. Further, $i$ is an integer ranging from 1 to M, representing an index at each point. Further, $j$ is an integer ranging from 1 to M, representing an index at a reference point. Further, $\Delta f_{1,i}$ represents a degree of change in the first feature at the i-th point when a reference point is j, and $\Delta f_{2,i}$ represents a degree of change in the second feature at the i-th point when a reference point is j.

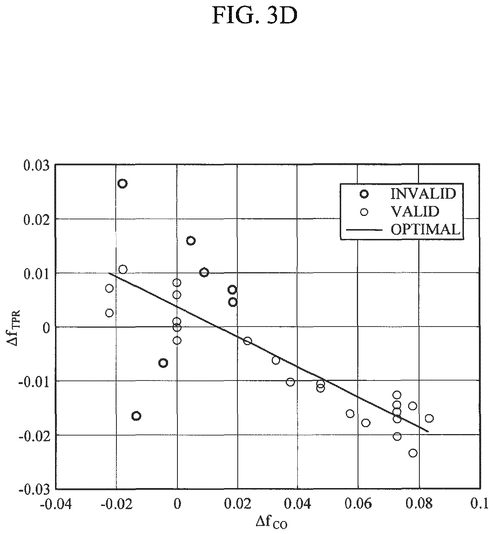

The calibration point determiner 220 may determine a valid point from among the plurality of points of the bio-signal, and determine a calibration point by combining the degree of change in the first feature at the valid point and the degree of change in the second feature at the valid point, as described above. In this case, as shown in FIG. 3D, the calibration point determiner 220 may acquire a correlation between a degree $\Delta f_{co}$ of change in the first feature and a degree $\Delta f_{TPR}$ of change in the second feature, and determine, based on the correlation, that a point greater than or equal to a predetermined threshold is a valid point.

In this case, the correlation may be calculated using various methods, such as Euclidean distance, Manhattan distance, cosine distance, Mahalanobis distance, Jaccard coefficient, extended Jaccard coefficient, Pearson's correlation coefficient, Spearman's correlation coefficient, and the like.

Based on the calibration point determiner 220 determining the calibration point, the calibrator 230 may determine the first feature and second feature at the determined calibration point as a first reference feature and a second reference feature, respectively, and calibrate a bio-information estimation model using the determined first and second reference features.

For example, and for convenience of description, a blood pressure estimation model is assumed to be defined by Equation 3 below. However, the exemplary embodiment is not limited thereto.

$$BP = SF \times (w_1 \Delta f_1 + w_2 \Delta f_2) + OFF \qquad \text{...Equation (3)}$$

Referring to Equation 3, $BP$ represents a blood pressure estimate at the time of estimation, and $\Delta f_1$ represents a degree of change in the first feature relative to the first reference feature at the time of estimation. $\Delta f_2$ represents a degree of change in the second feature relative to the second reference feature at the time of estimation. The first reference feature and the second reference feature represent, respectively, a feature associated with CO and a feature associated with TPR of the calibration point determined by the calibration point determiner 220. Further, $w_1$ and $w_2$ represent coupling coefficients, which may be values predefined through a preprocessing process. Further, $SF$ represents a scaling factor and may be a value that is predefined through a preprocessing process. In addition, the product of the amount of change in features ($w_1 \Delta f_1 + w_2 \Delta f_2$) and the scaling factor SF may be defined as the amount of change in blood pressure ΔBP. Further, $OFF$ is a value used for correcting the amount of change in blood pressure to acquire a blood pressure estimate and generally may be a cuff blood pressure measured at the time of calibration.

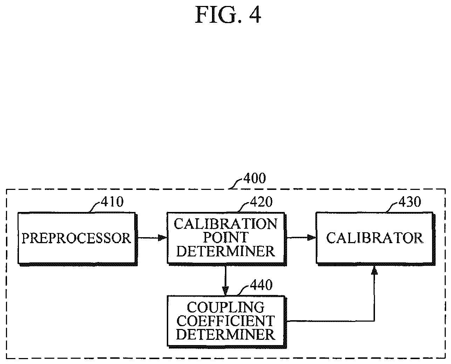

FIG. 4 is a block diagram illustrating another embodiment of a configuration of the processor of FIG. 1 according to an embodiment.

Referring to FIG. 4, the processor 400 may include a preprocessor 410, a calibration point determiner 420, a calibrator 430, and a coupling coefficient determiner 440.

Based on a bio-signal for calibration being received from a sensor 110, the preprocessor 410 may preprocess the received bio-signal. For example, the preprocessor 410 may perform noise removal, signal amplification, and the like, through normalization of a bio-signal, detrending, band-pass filtering, low-pass filtering, or the like.

In addition, the preprocessor 410 may detect a representative waveform from the bio-signal. As described above, the preprocessor 410 may detect a predetermined number of representative waveforms from a bio-signal. Alternatively, the preprocessor 410 may detect one or more representative waveforms from each of a plurality of bio-signals that are acquired during different time periods at predetermined time intervals during a current calibration, or from each of a plurality of bio-signals that were obtained during a previous calibration or at the time of blood pressure estimation.

The calibration point determiner 420 may extract two or more features from each of the representative waveforms, that is, each point of the bio-signal, as described above. In this case, the two or more features extracted from each point may include a first feature associated with CO and a second feature associated with TPR.

Also, the calibration point determiner 420 may determine a calibration point at which calibration is to be performed. The calibration point determiner 420 may determine one arbitrary point from among a plurality of points of the bio-signal as the calibration point.

For example, the calibration point determiner 420 may determine one point predetermined from among the plurality of points of the bio-signal as the calibration point. For instance, when a plurality of points are acquired from one bio-signal obtained during a current calibration, the first point, the intermediate point, and the like, in time may be determined as the calibration point. Alternatively, when a plurality of points are acquired from a plurality of bio-signals obtained at a plurality of times of the calibration, a point or an intermediate point obtained at the time of the current calibration that is the latest in time may be determined as the calibration point. However, the embodiment is not limited thereto.

In another example, as described above, each of the plurality of points of the bio-signal may be set as a reference point in turn, and a degree of change in the first feature at each point and a degree of change in the second feature at each point may be acquired and combined for each reference point and a point at which the combination result satisfies a predetermined criterion may be determined as the calibration point.

When the calibration point is determined, the coupling coefficient determiner 440 may calculate and normalize the degrees of changes in the first and second features at each point relative to the calibration point, and determine a coupling coefficient of a bio-information estimation model based on the calculated degrees of changes in the first and second features. In this case, the coupling coefficient may be a value used for coupling the degree of change in the first feature and the degree of change in the second feature based on the amount of change in features in the bio-information estimation model being acquired, and may be a parameter that is applied to at least to one of the degree of change in the first feature and the degree of change in the second feature to make them equal or similar to each other.

For example, the coupling coefficient determiner 440 may combine the degree of change in the first feature and the degree of change in the second feature at each point relative to the calibration point by applying the coupling coefficient to at least one of the degrees of changes in the first and second features, and determine a coupling coefficient that causes the combination results to satisfy a predetermined criterion.

Equation 4 below is one example by which the degrees of changes in the first and second features at each point are combined and a coupling coefficient is calculated. However, the embodiment is not limited thereto.

$$ SR_c = \sum_{i=1}^{M} \left| \left| \Delta f_{1,i} \right| - \alpha \times \left| \Delta f_{2,i} \right| \right| \qquad \text{...Equation (4)} $$

Herein, M represents the number of points of a bio-signal. Further, i represents an integer ranging from 1 to M, representing an index of each point. Further, c represents an index of a calibration point. Further, $\Delta f_{1,i}$ represents a degree of change in the first feature at the i-th point relative to the calibration point, and $\Delta f_{2,i}$ represents a degree of change in the second feature at the i-th point relative to the calibration point. Further, $\alpha$ represents a coupling coefficient applied to the degree of change in the second feature.

The coupling coefficient determiner 440 may determine a coupling coefficient $\alpha$ that makes a sum of residual have a minimum value in Equation 4 above. In this case, the coupling coefficient may be preset to have a value in a specific range (e.g., $0.8 \leq \alpha \leq 1.2$) based on a physiological or data-based analysis result. However, the range of the coupling coefficient may be varied based on at least one from among a user input, a user characteristic, an external environment characteristic, bio-information estimation history, a type of bio-information to be estimated, and computing performance metric of a device.

The coupling coefficient determiner 440 may determine a valid point from among a plurality of points of the bio-signal, and determine the coupling coefficient by combining the degrees of changes in the first and second features at the valid point, as described above. In this case, as shown in FIG. 3D, the coupling coefficient determiner 440 may obtain a correlation between the degree $\Delta f_{co}$ of change in the first feature and the degree $\Delta f_{TPR}$ of change in the second feature, and determine, based on the correlation, that a point greater than or equal to a predetermined threshold is a valid point.

A coupling coefficient $w_1$ of the degree of change in the first feature and a coupling coefficient $w_2$ of the degree

of change in the second feature are applied in the blood pressure estimation model of Equation 3 above to acquire the amount of change in the features ($w_1 \Delta f_1 + w_2 \Delta f_2$). In order to acquire such coupling coefficients, calibration blood pressure, e.g., cuff blood pressure information, may be obtained from various users through a preprocessing process. However, according to the present embodiment, a coupling coefficient that makes the degree of change in the first feature and the degree of change in the second feature equal or similar to each other may be acquired without using the calibration blood pressure.

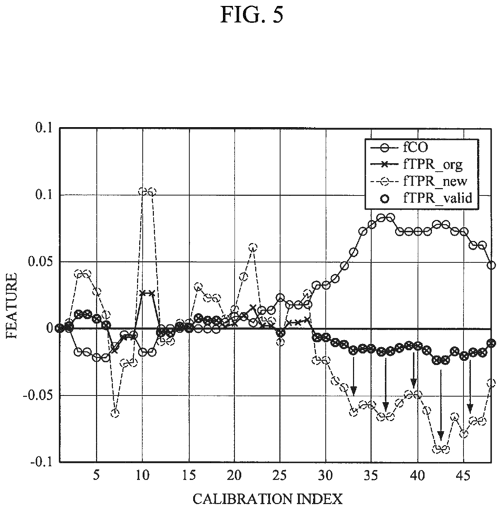

FIG. 5 is a graph showing a degree fCO of change in the first feature and degrees fTPR_org and fTPR_new of changes in the second feature relative to a determined calibration point which is a point at which a calibration index is 0. Referring to FIG. 5, fTPR_org represents an original degree of change in the second feature and fTPR_new represents a degree of change in the second feature that is obtained by applying the coupling coefficient $\alpha$ calculated as described above to fTPR_org. Further, fTPR_valid represents a degree of change in the second feature at the valid point determined as described above.

As shown in FIG. 5, the degree fTPR_new of change in the second feature that is obtained by applying the coupling coefficient $\alpha$ to the original degree fTPR_org of change in the second feature becomes similar to the degree fCO of change in the first feature. As such, a coupling coefficient that causes the degree of change in MAP to be close to zero at the time of calibration in a stable state may be acquired by making the degree of change in the second feature similar to the degree of change in the first feature and the coupling coefficient may be applied to the blood pressure estimation model, such as Equation 3 above, thereby improving accuracy in blood pressure measurement.

The calibrator 430 may apply the first and second features of the calibration point determined by the calibration point determiner 420 and the coupling coefficient determined by the coupling coefficient determiner 440 to the blood pressure estimation model, such as Equation 3 above.

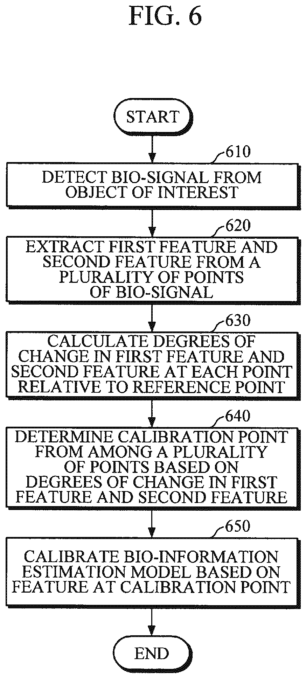

FIG. 6 is a flowchart illustrating a method of calibration according to an embodiment.

The method of FIG. 6 may be performed by the apparatus 100 of FIG. 1 for calibration. The calibration is described above with reference to FIGS. 1 and 2, and hence will be briefly described below.

First, the apparatus 100 may detect a bio-signal from an object of interest (operation 610). The apparatus 100 may monitor for an occurrence of a calibration event, and based on determining that a calibration event occurs, may detect a bio-signal for calibration. In this case, the calibration event may occur based on a user's input, a calibration period, a request from an external device, or the like. The apparatus 100 may measure a continuous bio-signal of an object of interest during a time period based on the occurrence of the calibration event. However, when a criterion for acquiring a bio-signal is preset, a plurality of bio-signals may be detected during a plurality of different time periods, or a bio-signal detected during a previous calibration may be further acquired in addition to a bio-signal detected from the object of interest.

Then, a first feature and a second feature may be extracted from a plurality of points of the bio-signal (operation 620). In this case, representative waveforms may be detected from a plurality of points on a time axis of the detected bio-signal, or one or more representative waveforms may be detected from each of the bio-signals, and a number of representative waveforms corresponding to the number of predetermined points may be detected. In addition, the first feature and the second feature may be extracted from each of the detected representative waveforms. In this case, the first feature may be a feature associated with CO, and the second feature may be a feature associated with TPR.

Then, degrees of change in the first feature and the second feature at each point relative to a reference point may be calculated (operation 630). For example, the apparatus 100 for calibration may set each of a plurality of points of the bio-signal as a reference point in turn, and calculate the degrees of change in the first feature and the second feature at each point relative to each reference point. In this case, abnormal points may be removed from the plurality of points based on a correlation between the degree of change in the first feature and the degree of change in the second feature, and then the apparatus 100 may calculate the degrees in change in the first feature and the second feature at each valid point.

Then, a calibration point may be determined from the plurality of points based on the calculated degrees of change in the first feature and the second feature at each point for each reference point (operation 640). For example, the degree of change in the first feature and the second feature at each point may be combined for each reference point and the reference point in which the combination result satisfies a predetermined criterion, for example, a point where the sum of residual between the degree of change in the first feature and the degree of change in the second feature has the minimum value, as shown in Equation 2, may be determined as the calibration point.

Then, a bio-information estimation model may be calibrated based on the first feature and the second feature at the determined calibration point (operation 650). For example, the first feature and the second feature at the calibration point may be set as a first reference point and a second reference point, respectively, to be used for acquiring degrees of change in the first feature and the second feature at the time of estimation of the blood pressure as shown in Equation 3.

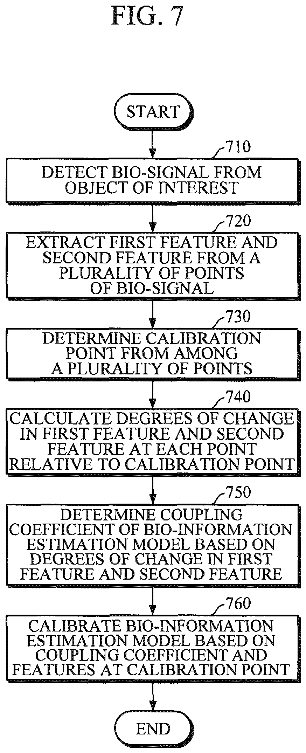

FIG. 7 is a flowchart illustrating a method of calibration according to another embodiment.

The method of FIG. 7 may be performed by the apparatus 100 of FIG. 1 for calibration. The calibration is described above with reference to FIGS. 1 and 4 and hence will be briefly described below.

First, the apparatus 100 for calibration may detect a bio-signal from an object of interest (operation 710). The apparatus 100 may detect a continuous bio-signal of the object of interest during a time period, or may detect a plurality of bio-signals during a plurality of different time periods based on a setting, or may further acquire a bio-signal detected from the object of interest during a previous calibration.

Then, a first feature and a second feature may be extracted from a plurality of points of the bio-signal (operation 720).

Then, an arbitrary point among the plurality of points of the bio-signal may be determined as a calibration point (operation 730). For example, a point (e.g., an initial point) that is preset from the plurality of points may be determined as a calibration point. Alternatively, each of the plurality of points may be set as a reference point in turn and a reference point where a combination of changes of the first feature and the second feature at each point relative to each reference point satisfies a predetermined criterion may be determined as a calibration point.

Then, degrees of change in the first feature and the second feature at each point relative to the calibration point may be calculated (operation 740), and a coupling coefficient of a bio-information estimation model may be determined based on the calculated degrees of change in the first feature and the second feature (operation 750). For example, as described by taking Equation 4 as an example, a coupling coefficient may be applied to at least one of the degree of change in the first feature and the degree of change in the second feature to obtain the sum of residual between the degree of change in the first feature and the degree of change in the second feature, and a coupling coefficient that causes the sum of residual to have the minimum value may be determined. In this case, the range of the coupling coefficient may be preset.

Then, the bio-information estimation model may be calibrated based on the first feature and the second feature at the calibration point determined in operation 730 and the coupling coefficient determined in operation 750 (operation 760).

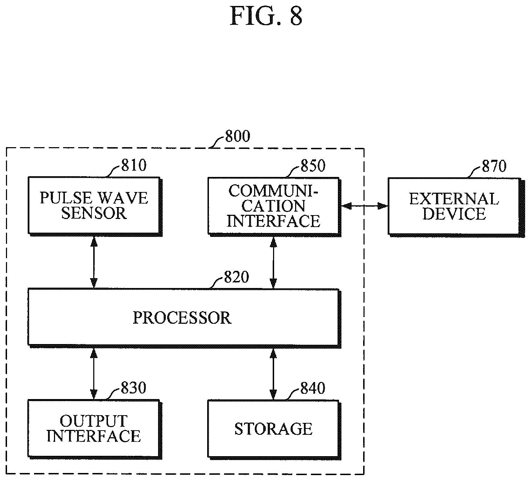

FIG. 8 is a block diagram illustrating an apparatus for estimating bio-information according to an embodiment.

Referring to FIG. 8, the apparatus 800 for estimating bio-information may include a pulse wave sensor 810, a processor 820, an output interface 830, a storage 840, and a communication interface 850.

The pulse wave sensor 810 may detect a pulse wave signal including a PPG signal from an object of interest of a user. The pulse wave sensor 810 may include a light source configured to emit light towards the object of interest, and a detector configured to detect light absorbed, scattered, or reflected by the object of interest. The light source may include one or more LEDs, laser diodes (LDs), or phosphors. The detector may include one or more photodiodes, photo transistors (PTrs), image sensors (e.g., CMOS image sensors), or the like.

The processor 820 may be electrically connected to the pulse wave sensor 810. The processor 820 may receive the pulse wave signal by controlling the pulse wave sensor 810 for calibration or bio-information estimation, and use the pulse wave signal to calibrate a bio-information estimation model or to estimate bio-information. Here, the bio-information may include blood pressure, vascular age, a degree of arteriosclerosis, an aortic pressure waveform, vascular compliance, a stress index, a degree of fatigue, and the like. However, the bio-information is not limited thereto.

For example, the processor 820 may perform a calibration function of the above-described apparatus 100 for calibration. The processor 820 may monitor for an occurrence of a calibration event, and detect one or more first pulse wave signals for calibration by controlling the pulse wave sensor 810 based on detecting an occurrence of a calibration event.

In addition, the processor 820 may acquire a calibration point and/or a coupling coefficient using the one or more first pulse wave signals. As described above, each of a plurality of points of the first pulse wave signal may be set as a reference point and a point where the sum of residual between a degree of change in a first feature and a degree of change in a second feature at each point has a minimum value may be determined as the calibration point. In addition, a coupling coefficient that causes the sum of residual between the changes of the first feature and second feature at each point has a minimum value with respect to the determined calibration point may be calculated.

In another example, the processor 820 may control the communication interface 850 to communicate with an external device 870 based on detecting an occurrence of a calibration event. The processor 820 may request the external device 870 to perform calibration by transmitting a first pulse wave signal detected by the pulse wave sensor 810, and may receive, from the external device 870, information regarding a coupling coefficient and/or features at a calibration point which are acquired by the external device 870 which performs a calibration as described above.

The processor 820 may store the acquired coupling coefficient and/or the features at the calibration point in the storage 840, and update a bio-information estimation model stored in the storage 840.

The processor 820 may control the pulse wave sensor 810 to detect a second pulse wave signal for estimating bio-information based on detecting an occurrence of a bio-information estimation event, and may extract a first feature and a second feature by analyzing a waveform of the second pulse wave signal. Also, the processor 820 may acquire a first feature, a second feature, and a coupling coefficient, and the bio-information estimation model at the calibration point by using the storage 840. The processor 820 may calculate degrees of change in the first and second features extracted from the second pulse wave signal based on the first feature and the second feature at the calibration point, and acquire a degree of change in a feature by combining the degree of change in the first feature and the degree of change in the second feature based on the coupling coefficient. In this case, the coupling coefficient may be a fixed value predefined through a preprocessing process on a plurality of users or may be a value obtained by the processor 820 as described above. The processor 820 may estimate bio-information by applying the acquired degree of change in the feature to the bio-information estimation model such as Equation 3.

The output interface 830 may output a processing result of the processor 820. The output interface 830 may include a visual output component, such as a display, an audio output component, such as a speaker, a haptic component which provides information such as vibration or tactile sensation, and may output information for the user by utilizing the visual/non-visual output component.

For example, the output interface 830 may divide a display into a first section and a second section, output a bio-information estimation result, a bio-information estimation history, and the like to the first section, and output the first pulse wave signal, the second pulse wave signal, and the like to the second section. In this case, the display may include a touch screen capable of receiving a touch input. Based on the user selecting a bio-information estimation result at a specific point in time from the bio-information estimation history, the output interface 830 may output detailed information of the corresponding time point to the second section. However, this is merely an example and the embodiment is not limited thereto.

The storage 840 may store information for bio-information estimation. For example, the storage 840 may store a pulse wave signal detected by the pulse wave sensor 810 and/or the processing result of the processor 820. In addition, the storage 840 may store user characteristic information, such as the user's age, sex, health status, and the like, the bio-information estimation model, and reference information, such as the number of points to be acquired from a bio-signal, a window size, a pulse wave signal measurement duration, and the like.

The communication interface 850 may access a communication network under the control of the processor 820 or communicate with an external device connected to a currently accessed communication network. In this case, the external device may include, but is not limited to, an information processing device, such as a cuff-blood pressure monitor, a calibration device, a smartphone, a tablet PC, a desktop PC, a notebook PC, and the like.

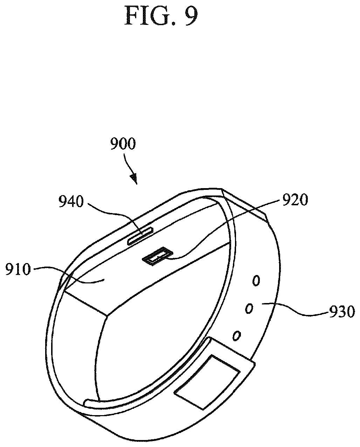

FIG. 9 is a diagram illustrating a wearable device to be worn on a wrist according to an embodiment. The embodiments of the above-described apparatus 100 for calibration or the apparatus 800 for estimating bio-information may be mounted in a smart watch or a smart band-type wearable device to be worn on a wrist. However, the embodiments are not limited thereto.

Referring to FIG. 9, the wearable device 900 may include a main body 910, a pulse wave sensor 920, a strap 930, and an input interface 940.

The main body 910 may be configured to have various shapes, have various modules mounted inside or on an outer surface thereof for performing the above-described calibration function or bio-information estimation function, and have modules for performing various other functions (e.g., a watch function and an alarm function). A battery may be equipped in the main body 910 or the strap 930 to supply power to various modules in the device 900.

The strap 930 may be connected to the main body 910. The strap 930 may be flexibly configured to be bent to wrap around a wrist of a user. The strap 930 may be configured in a form that is separated from the user's wrist or in a band form that is not separated. The strap 930 may be filled with air or an air bag so as to have elasticity according to a change in pressure applied to the wrist, and may transmit a pressure change of the wrist to the main body 910.

A pulse wave sensor 920 may be mounted in the main body 910 to detect a pulse wave signal. The pulse wave sensor 920 may be mounted on one surface of the main body 910 which is in contact with the user's wrist when the main body 910 is worn on the wrist of the user. The pulse wave sensor 920 may include a light source configured to emit light towards the wrist of the user, and a detector configured to detect light scattered or reflected by the in-vivo tissues, such as a skin surface, blood vessels, or the like.

In addition, a processor may be mounted inside the main body 910 and may be electrically connected to the components of the wearable device 900 to control the components.

The processor may control the pulse wave sensor 920 to detect a pulse wave signal based on detecting an occurrence of a calibration event, and may perform calibration based on the detected pulse wave signal. Also, based on detecting an occurrence of a bio-information estimation event, the processor may control the pulse wave sensor 920 to detect the pulse wave signal, and may estimate bio-information based on the detected pulse wave signal and a calibrated bio-information estimation model. The calibration and the bio-information estimation are described above in detail and hence detailed descriptions thereof will not be reiterated.

A display may be mounted on a front surface of the main body 910 and may be a touch panel including a touch screen capable of receiving a touch input. The display may receive a touch input of the user and forward the touch input to the processor and may output a processing result of the processor. For example, the display may display a bio-information estimate and display additional information, such as a bio-information estimation history, a change of health status, a warning, and the like.

The storage may be mounted inside the main body 910 to store the processing results of the processor and other information. In this case, in addition to information related to bio-information estimation, the storage may store information related to other functions of the wearable device 900.

Also, an input interface 940 may be mounted in the main body 910 to receive an input of the user and forward the input to the processor. The input interface 940 may include a power button to enable the user to input an instruction for power on/off of the wearable device 900.

In addition, a communication interface may be mounted inside the main body 910 to communicate with an external device. The communication interface may transmit the bio-information estimation result to the external device such as, for example, a mobile terminal of the user, such that the bio-information estimation result can be output via an output module of the external device or be stored in a storage of the external device. Moreover, the communication interface may receive information for supporting various other functions performed by the wearable device to the external device.

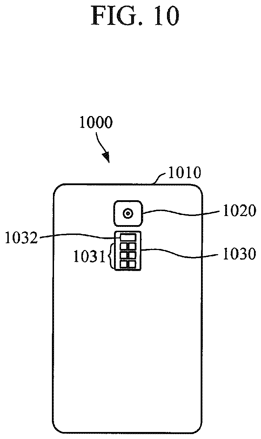

FIG. 10 is a diagram illustrating a smart device to which the embodiments of the above-described apparatus 100 for calibration or the apparatus 800 for estimating bio-information are applied according to an embodiment. In this case, the smart device may be a smartphone or a tablet PC, but is not limited thereto.

Referring to FIG. 10, a pulse wave sensor 1030 may be mounted on one surface of a main body 1010 of the smart device 1000. The pulse wave sensor 1030 may include one or more light sources 1031 and a detector 1032. The pulse wave sensor 1030 may be mounted on a rear surface of the main body 1010 as shown in FIG. 10, but is not limited thereto. The pulse wave sensor 1030 may be configured to be coupled to a fingerprint sensor or a touch panel on the front surface.

In addition, a force sensor may be mounted inside the main body 1010 and may detect a force applied by a finger or the like of the user in contact with the pulse wave sensor and forward the detected force to the processor.

A display may be mounted on the front surface of the main body 1010. The display may visually output a bio-information estimation result and the like. The display may include a touch panel, may receive information input through the touch panel, and may forward the information to the processor.

An image sensor 1020 may be mounted in the main body 1010. The image sensor 1020 may capture an image of a finger of the user based on the user being within proximity of the pulse wave sensor 1030 to measure a pulse wave signal, and may forward the image to the processor. In this case, the processor may recognize a relative position of the finger compared to a position of the pulse wave sensor 1030 from the image of the finger and provide the relative position information of the finger to the user through the display so that the user can be instructed to correctly bring his/her finger into contact with the pulse wave sensor 1030.

The processor may calibrate a bio-information estimation model or estimate bio-information using the pulse wave signal detected by the pulse wave sensor 1030. The calibration or the bio-information estimation is described above in detail and hence the detailed description thereof will be omitted.

The current embodiments can be implemented as computer readable code stored in a non-transitory computer readable medium. Code and code segments constituting the computer program can be inferred by a person skilled in the art. The computer readable medium includes all types of record media in which computer readable data are stored. Examples of the computer readable medium include a ROM, a RAM, a CD-ROM, a magnetic tape, a floppy disk, and an optical data storage. Further, the computer readable medium may be implemented in the form of a carrier wave such as Internet transmission. In addition, the computer readable medium may be distributed to computer systems over a network, in which computer readable code may be stored and executed in a distributed manner.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

**Claims**

1. A computer-implemented method for improving the accuracy of a bio-information measurement, wherein the bio-information includes at least one of blood pressure, vascular age, a degree of arteriosclerosis, an aortic pressure waveform, vascular compliance, a stress index, and a degree of fatigue, the method comprising:

   obtaining (610) a bio-signal associated with an object of interest, wherein the bio-signal includes a pulse wave signal;
   extracting (620) a first feature from each of a plurality of points of the pulse wave signal, wherein the first feature has a high degree of correlation with the bio-information;
   extracting a second feature from each of the plurality of points of the pulse wave signal, wherein the second feature has a high degree of correlation with the bio-information;

identifying (630) a first degree of change in the first feature at each point relative to a first feature at a reference point;

identifying a second degree of change in the second feature at each point relative to a second feature at the reference point, wherein the identifying of the first degree of change in the first feature and the second degree of change in the second feature respectively comprise setting each of the plurality of points as the reference point in turn and calculating the first degree of change in the first feature and the second degree of change in the second feature at each point relative to each reference point;

determining (640) a calibration point of a bio-information estimation model from among the plurality of points of the bio-signal based on the first degree of change in the first feature and the second degree of change in the second feature, wherein the determining of the calibration point comprises calculating a sum of residual between the first degree of change in the first feature and the second degree of change in the second feature at each point for each reference point and determining a reference point at which the calculated sum of residual has a minimum value as the calibration point;

updating (650) the bio-information estimation model based on the first feature and the second feature at the determined calibration point; and

estimating the bio-information based on an obtained pulse wave signal and the updated bio-information estimation model.

2. The computer-implemented method of claim 1, wherein the determining of the calibration point comprises determining two or more valid points from among the plurality of points and determining the calibration point from among the determined valid points.

3. The computer-implemented method of claim 2, wherein the determining of the calibration point comprises determining the valid points based on a correlation between the first degree of change in the first feature and the second degree of change in the second feature at each point.

4. The computer-implemented method of claim 1 further comprising:

identifying (740) a first degree of change in a first feature at each point relative to a first feature at the determined calibration point;

identifying a second degree of change in a second feature at each point relative to a second feature at the determined calibration point; and

determining (750) a coupling coefficient of the bio-information estimation model based on the first degree of change in the first feature and the second degree of change in the second feature.

5. The computer-implemented method of claim 4,

wherein the determining of the coupling coefficient comprises determining the coupling coefficient such that a result of combining the first degree of change in the first feature and the second degree of change in the second feature at each point by applying the coupling coefficient to one of the first degree of change in the first feature and the second degree of change in the second feature satisfies a predetermined criterion, or

wherein the determining of the coupling coefficient comprises determining the coupling coefficient such that a sum of residual between the first degree of change in the first feature and the second degree of change in the second feature has a minimum value, or

wherein the determining of the coupling coefficient comprises determining two or more valid points from among the plurality of points and determining the coupling coefficient based on respective degrees of change in a first feature and a second feature at the determined valid points.

6. An apparatus (100) for measuring bio-information with improved accuracy, the apparatus comprising:

a sensor (110) configured to detect one or more bio-signals from an object of interest, wherein each of the one or more bio-signals includes a pulse wave signal; and

a processor (120) configured to perform the method of claim 1.

7. A computer-readable medium having instructions that, when performed by a processor, causes the processor to perform the method of claim 1.

**Patentansprüche**

1. Ein computerimplementiertes Verfahren zur Verbesserung der Genauigkeit einer Bioinformationsmessung, wobei die Bioinformation mindestens eines der folgenden Elemente umfasst: Blutdruck, Gefäßalter, Grad der Arteriosklerose, Aorten-Druckwellenform, Gefäßnachgiebigkeit, Stressindex und Ermüdungsgrad, wobei das Verfahren Folgendes umfasst:

Erhalten (610) eines Biosignals, das mit einem Objekt von Interesse verbunden ist, wobei das Biosignal ein Pulswellensignal enthält;

Extrahieren (620) eines ersten Merkmals aus jedem einer Vielzahl von Punkten des Pulswellensignals, wobei das erste Merkmal einen hohen Korrelationsgrad mit den Bio-Informationen aufweist;

Extrahieren eines zweiten Merkmals aus jedem der Vielzahl von Punkten des Pulswellensignals, wobei das zweite Merkmal einen hohen Korrelationsgrad mit der Bio-Information aufweist;

Identifizierung (630) eines ersten Grades der Veränderung des ersten Merkmals an jedem Punkt relativ zu einem ersten Merkmal an einem Referenzpunkt;

Identifizieren eines zweiten Grades der Veränderung in dem zweiten Merkmal an jedem Punkt relativ zu einem zweiten Merkmal an dem Referenzpunkt, wobei das Identifizieren des ersten Grades der Veränderung in dem ersten Merkmal und des zweiten Grades der Veränderung in dem zweiten Merkmal jeweils das Festlegen jedes der Vielzahl von Punkten als den Referenzpunkt der Reihe nach und das Berechnen des ersten Grades der Veränderung in dem ersten Merkmal und des zweiten Grades der Veränderung in dem zweiten Merkmal an jedem Punkt relativ zu jedem Referenzpunkt umfasst;

Bestimmen (640) eines Kalibrierungspunktes eines Bioinformations-Schätzmodells aus der Vielzahl von Punkten des Biosignals auf der Grundlage des ersten Grades der Änderung in dem ersten Merkmal und des zweiten Grades der Änderung in dem zweiten Merkmal, wobei das Bestimmen des Kalibrierungspunktes das Berechnen einer Restsumme zwischen dem ersten Grad der Änderung in dem ersten Merkmal und dem zweiten Grad der Änderung in dem zweiten Merkmal an jedem Punkt für jeden Referenzpunkt und das Bestimmen eines Referenzpunktes, an dem die berechnete Restsumme einen minimalen Wert hat, als den Kalibrierungspunkt umfasst;

Aktualisieren (650) des Bioinformations-Schätzmodells auf der Grundlage des ersten Merkmals und des zweiten Merkmals an dem bestimmten Kalibrierungspunkt; und

Schätzung der Bio-Informationen auf der Grundlage eines erhaltenen Pulswellensignals und des aktualisierten Modells zur Schätzung der Bio-Informationen.

2. Das computerimplementierte Verfahren nach Anspruch 1, wobei das Bestimmen des Kalibrierungspunkts das Bestimmen von zwei oder mehr gültigen Punkten aus der Vielzahl von Punkten und das Bestimmen des Kalibrierungspunkts aus den bestimmten gültigen Punkten umfasst.

3. Das computerimplementierte Verfahren nach Anspruch 2, wobei das Bestimmen des Kalibrierungspunktes das Bestimmen der gültigen Punkte auf der Grundlage einer Korrelation zwischen dem ersten Grad der Veränderung des ersten Merkmals und dem zweiten Grad der Veränderung des zweiten Merkmals an jedem Punkt umfasst.

4. Das computerimplementierte Verfahren nach Anspruch 1 ferner umfassend:

Identifizierung (740) eines ersten Grades der Veränderung eines ersten Merkmals an jedem Punkt relativ zu einem ersten Merkmal am bestimmten Kalibrierungspunkt;

Identifizierung eines zweiten Grades der Veränderung eines zweiten Merkmals an jedem Punkt relativ zu einem zweiten Merkmal am bestimmten Kalibrierungspunkt; und

Bestimmen (750) eines Kopplungskoeffizienten des Bioinformationsschätzungsmodells auf der Grundlage des ersten Grades der Veränderung des ersten Merkmals und des zweiten Grades der Veränderung des zweiten Merkmals.

5. Das computerimplementierte Verfahren nach Anspruch 4,

wobei das Bestimmen des Kopplungskoeffizienten das Bestimmen des Kopplungskoeffizienten derart umfasst, dass ein Ergebnis des Kombinierens des ersten Grades der Änderung in dem ersten Merkmal und des zweiten Grades der Änderung in dem zweiten Merkmal an jedem Punkt durch Anwenden des Kopplungskoeffizienten auf einen von dem ersten Grad der Änderung in dem ersten Merkmal und dem zweiten Grad der Änderung in dem zweiten Merkmal ein vorbestimmtes Kriterium erfüllt, oder

wobei das Bestimmen des Kopplungskoeffizienten das Bestimmen des Kopplungskoeffizienten derart umfasst, dass eine Summe von Residuen zwischen dem ersten Grad der Veränderung des ersten Merkmals und dem zweiten Grad der Veränderung des zweiten Merkmals einen Minimalwert hat, oder
wobei das Bestimmen des Kopplungskoeffizienten das Bestimmen von zwei oder mehr gültigen Punkten aus der Vielzahl von Punkten und das Bestimmen des Kopplungskoeffizienten auf der Grundlage von jeweiligen Änderungsgraden in einem ersten Merkmal und einem zweiten Merkmal an den bestimmten gültigen Punkten umfasst.

6. Eine Vorrichtung (100) zum Messen von Bio-Informationen mit verbesserter Genauigkeit, wobei die Vorrichtung umfasst:

   einen Sensor (110), der so konfiguriert ist, dass er ein oder mehrere Biosignale von einem Objekt von Interesse erfasst, wobei jedes des einen oder der mehreren Biosignale ein Pulswellensignal enthält; und
   einen Prozessor (120), der zur Durchführung des Verfahrens nach Anspruch 1 konfiguriert ist.

7. Ein computerlesbares Medium mit Anweisungen, die, wenn sie von einem Prozessor ausgeführt werden, den Prozessor veranlassen, das Verfahren nach Anspruch 1 durchzuführen.

**Revendications**

1. Procédé mis en oeuvre par ordinateur pour améliorer la précision d'une mesure de bio-information, dans lequel la bio-information comprend au moins l'un parmi une pression artérielle, un âge vasculaire, un degré d'artériosclérose, une forme d'onde de pression aortique, une compliance vasculaire, un indice de stress, et un degré de fatigue, le procédé comprenant les étapes consistant à :

   obtenir (610) un bio-signal associé à un objet d'intérêt, le bio-signal comprenant un signal d'onde de pouls ;
   extraire (620) une première caractéristique à partir de chacun d'une pluralité de points du signal d'onde de pouls, la première caractéristique ayant un degré élevé de corrélation avec la bio-information ;
   extraire une seconde caractéristique à partir de chacun de la pluralité de points du signal d'onde de pouls, la seconde caractéristique ayant un degré élevé de corrélation avec la bio-information ;
   identifier (630) un premier degré de changement dans la première caractéristique à chaque point par rapport à une première caractéristique à un point de référence ;
   identifier un second degré de changement dans la seconde caractéristique à chaque point par rapport à une seconde caractéristique au point de référence, dans lequel l'identification du premier degré de changement dans la première caractéristique et du second degré de changement dans la seconde caractéristique comprend respectivement l'établissement de chacun de la pluralité de points en tant que point de référence à tour de rôle et le calcul du premier degré de changement dans la première caractéristique et du second degré de changement dans la seconde caractéristique à chaque point par rapport à chaque point de référence ;
   déterminer (640) un point d'étalonnage d'un modèle d'estimation de bio-information parmi la pluralité de points du bio-signal sur la base du premier degré de changement dans la première caractéristique et du second degré de changement dans la seconde caractéristique, la détermination du point d'étalonnage comprenant le calcul d'une somme de résidu entre le premier degré de changement dans la première caractéristique et le second degré de changement dans la seconde caractéristique à chaque point pour chaque point de référence et la détermination d'un point de référence au niveau duquel la somme calculée de résidu a une valeur minimale en tant que point d'étalonnage ;
   mettre à jour (650) le modèle d'estimation de bio-information sur la base de la première caractéristique et de la seconde caractéristique au point d'étalonnage déterminé ; et
   estimer la bio-information sur la base d'un signal d'onde de pouls obtenu et du modèle d'estimation de bio-information mis à jour.

2. Procédé mis en oeuvre par ordinateur selon la revendication 1, dans lequel la détermination du point d'étalonnage comprend la détermination de deux ou plus de deux points valides parmi la pluralité de points et la détermination du point d'étalonnage parmi les points valides déterminés.

3. Procédé mis en oeuvre par ordinateur selon la revendication 2, dans lequel la détermination du point d'étalonnage comprend la détermination des points valides sur la base d'une corrélation entre le premier degré de changement dans la première caractéristique et le second degré de changement dans la seconde caractéristique à chaque point.

**4.** Procédé mis en oeuvre par ordinateur selon la revendication 1, comprenant en outre les étapes consistant à :

identifier (740) un premier degré de changement dans une première caractéristique à chaque point par rapport à une première caractéristique au point d'étalonnage déterminé ;
identifier un second degré de changement dans une seconde caractéristique à chaque point par rapport à une seconde caractéristique au point d'étalonnage déterminé ; et
déterminer (750) un coefficient de couplage du modèle d'estimation de bio-information sur la base du premier degré de changement dans la première caractéristique et du second degré de changement dans la seconde caractéristique.

**5.** Procédé mis en oeuvre par ordinateur selon la revendication 4,

dans lequel la détermination du coefficient de couplage comprend la détermination du coefficient de couplage de sorte qu'un résultat de la combinaison du premier degré de changement dans la première caractéristique et du second degré de changement dans la seconde caractéristique à chaque point en appliquant le coefficient de couplage à l'un parmi le premier degré de changement dans la première caractéristique et le second degré de changement dans la seconde caractéristique satisfait à un critère prédéterminé, ou
dans lequel la détermination du coefficient de couplage comprend la détermination du coefficient de couplage de sorte qu'une somme de résidu entre le premier degré de changement dans la première caractéristique et le second degré de changement dans la seconde caractéristique a une valeur minimale, ou
dans lequel la détermination du coefficient de couplage comprend la détermination de deux ou plus de deux points valides parmi la pluralité de points et la détermination du coefficient de couplage sur la base de degrés respectifs de changement dans une première caractéristique et une seconde caractéristique aux points valides déterminés.

**6.** Appareil (100) pour mesurer une bio-information avec une précision améliorée, l'appareil comprenant :

un capteur (110) configuré pour détecter un ou plusieurs bio-signaux à partir d'un objet d'intérêt, dans lequel chacun des un ou plusieurs bio-signaux comprend un signal d'onde de pouls ; et
un processeur (120) configuré pour effectuer le procédé de la revendication 1.

**7.** Support lisible par ordinateur contenant des instructions qui, lorsqu'elles sont effectuées par un processeur, amènent le processeur à effectuer le procédé de la revendication 1.

FIG. 1

FIG. 2

200

210

PREPROCESSOR

220

CALIBRATION
POINT
DETERMINER

230

CALIBRATOR

FIG. 3A

FIG. 3B

EP 3 753 484 B1

FIG. 3C

FIG. 3D

FIG. 4

400

| 410 | 420 | 430 |
| PREPROCESSOR | CALIBRATION POINT DETERMINER | CALIBRATOR |

440
COUPLING COEFFICIENT DETERMINER

FIG. 5

# FIG. 6

START

DETECT BIO-SIGNAL FROM
OBJECT OF INTEREST — 610

EXTRACT FIRST FEATURE AND
SECOND FEATURE FROM A
PLURALITY OF POINTS
OF BIO-SIGNAL — 620

CALCULATE DEGREES OF
CHANGE IN FIRST FEATURE AND
SECOND FEATURE AT EACH POINT
RELATIVE TO REFERENCE POINT — 630

DETERMINE CALIBRATION POINT
FROM AMONG A PLURALITY
OF POINTS BASED ON
DEGREES OF CHANGE IN FIRST
FEATURE AND SECOND FEATURE — 640

CALIBRATE BIO-INFORMATION
ESTIMATION MODEL BASED ON
FEATURE AT CALIBRATION POINT — 650

END

# FIG. 7

START

↓

710

DETECT BIO-SIGNAL FROM
OBJECT OF INTEREST

↓

720

EXTRACT FIRST FEATURE AND
SECOND FEATURE FROM A
PLURALITY OF POINTS
OF BIO-SIGNAL

↓

730

DETERMINE CALIBRATION
POINT FROM AMONG
A PLURALITY OF POINTS

↓

740

CALCULATE DEGREES OF CHANGE
IN FIRST FEATURE AND SECOND
FEATURE AT EACH POINT
RELATIVE TO CALIBRATION POINT

↓

750

DETERMINE COUPLING
COEFFICIENT OF BIO-INFORMATION
ESTIMATION MODEL BASED ON
DEGREES OF CHANGE IN FIRST
FEATURE AND SECOND FEATURE

↓

760

CALIBRATE BIO-INFORMATION
ESTIMATION MODEL BASED ON
COUPLING COEFFICIENT AND
FEATURES AT CALIBRATION POINT

↓

END

# FIG. 8

FIG. 9

FIG. 10

**EP 3 753 484 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3130280 A1